# EUROPEAN PATENT SPECIFICATION

(11) **EP 4 644 106 B1**
(45) Date of publication and mention of the grant of the patent: **11.03.2026**
(21) Application number: 24174079.4
(22) Date of filing: 03.05.2024
(51) Int. Cl.: B30B 11/00, G01N 21/3563, G01N 21/95, G01N 33/15, B30B 15/08

(54) **CALIBRATION DEVICE AND METHOD FOR CALIBRATING A TABLET SENSOR FOR A TABLET PRESS**
KALIBRIERVORRICHTUNG UND VERFAHREN ZUM KALIBRIEREN EINES TABLETTENSENSORS FÜR EINE TABLETTENPRESSE
DISPOSITIF D'ÉTALONNAGE ET PROCÉDÉ D'ÉTALONNAGE D'UN CAPTEUR DE COMPRIMÉS POUR UNE PRESSE À COMPRIMÉS

(43) Date of publication of application: 05.11.2025
(73) Proprietor: Fette Compacting GmbH, 21493 Schwarzenbek (DE)
(72) Inventor: Krumme, Markus, 4123 Allschwil (CH); Lüdemann, Stefan, 21035 Hamburg (DE); Kolbe, Sven, 21514 Büchen (DE)
(74) Representative: Hauck Patent- und Rechtsanwälte PartmbB

(56) References cited:
- JP-A- H11 137 641
- LAI C K ET AL: "Nondestructive and on-line monitoring of tablets using light-induced fluorescence technology", AAPS PHARMSCI, XX, XX, 1 January 2003 (2003-01-01), pages 1 - 10, XP002335117
- DOMIKE R ET AL: "Light induced fluorescence for predicting API content in tablets: Sampling and error", INTERNATIONAL JOURNAL OF PHARMACEUTICS, ELSEVIER, vol. 391, no. 1-2, 31 May 2010 (2010-05-31), pages 13 - 20, XP027018864, ISSN: 0378-5173, [retrieved on 20100213]

## Description

The invention relates to a calibration device and method for calibrating a tablet sensor for a tablet press, said tablet sensor being configured to measure a content property of tablets.

A tablet sensor for measuring a content property of tablets produced in a tablet press, in particular a content of an active pharmaceutical ingredient (API) is known for example from EP 1 568 480 B1. Apart from one or more APIs, tablets produced in tablet presses usually further comprise one or more excipients. The overall weight of tablets produced in the tablet press is known and can for example be measured. Based on measuring data from the tablet sensor regarding for example the content of an API in the tablet, it can be evaluated whether the API content and weight of tablets produced in the tablet press is within specified parameters.

The tablet sensor disclosed in EP 1 568 480 B1 is a NIR or LIF sensor which is arranged in the compression chamber of the tablet press such that it obtains measuring data of tablets produced after the upper punches of the tablet press have been retracted from the cavities of the die disk. The tablet sensor is stationary disposed above the die plate such that tablets still positioned within a cavity of the die disk pass through the measuring field of the tablet sensor. Another example is disclosed by LAI C.K. et al, "Nondestructive and on-line monitoring of tablets using light-induced fluorescence technology",AAPS PHARMSCI, XX, XX, 1 January 2003 (2003-01-01), pages 1-10.

There is a need to calibrate tablet sensors for example of the type disclosed in EP 1 568 480 B 1, in particular when a new product mixture is to be compressed in a tablet press. More specifically, it is necessary to obtain measuring data with the tablet sensor for tablets produced with the respective product mixture. The calibration can be done on basis of known content properties of the different ingredients of the product mixture. In order to calibrate the tablet sensor arranged within the compression chamber of the tablet press, the tablet press must be placed in regular production operating condition for continuous production with the new product mixture such that tablets produced in the tablet press pass through the measuring field of the tablet sensor for calibration. For tablet presses of the kind concerned, in particular high-capacity rotary tablet presses, a considerable amount of product must be filled into the filling device of the tablet press such that the tablet press can reach regular production conditions. Also, a considerable amount of tablets must be produced during the calibration procedure. The product mixture used as well as the tablets produced are waste after the calibration procedure is finished. Especially in early product development phases of pharmaceutical tablets the product mixture is particularly expensive and difficult to make available. In usual rotary tablet presses the minimum amount of product material for reaching production conditions is several kilograms. Of course, each tablet produced can only be used once for calibration purposes.

In order to reach normal production conditions of the tablet press it is further necessary to adjust all production parameters of the tablet press, in particular for a new product. This relates for example to the adjustment of the filling station or the pressing station as well as the adjustment of rotational speeds of the rotor and for example filling paddles in filling devices. This is a complex and time-consuming task and further increases costs of the calibration process. Furthermore, the measuring data obtained by the tablet sensor during calibration depends on a plurality of adjustment parameters and other boundary conditions. For the calibration, in particular the creation of a measuring model for the product mixture in question, all of these parameters and boundary conditions have to be controlled and documented. Any mistakes made in this regard have considerable effects on the calibration of the tablet sensor.

In order to avoid at least some of the above explained disadvantages a static measurement of tablets being manually placed in the measuring field of the tablet sensor has been suggested. In this way, the use of a large amount of production material and the other above explained disadvantages associated with calibrating the tablet sensor in continuous production operation of the tablet press can be avoided.

However, the relative movement between a tablet and the tablet sensor in normal production of the tablet press has a considerable influence on the measuring results. During production, a tablet enters the measuring field of the tablet sensor, rotates through the measuring field and exits the measuring field again. The tablet sensor thus usually obtains measuring data not only on a specific point of the tablet surface, but rather along a trajectory over the surface of the tablet passing through the measuring field. Any influences of this dynamic measurement cannot be considered during a static calibration process, where the tablet sensor only obtains measuring data from a specific point on the surface of the tablet. Also, the influence of different rotational speeds of the rotor of the tablet press on the obtained measuring data cannot be taken into account. Calibration of the tablet sensor on basis of a static measurement has thus led to considerable deviations with regard to the actual measuring data obtained during a subsequent production process. This makes complex and difficult adjustments necessary during production.

Another issue in the prior art relates to the position of the tablet sensor in the compression chamber of a tablet press. Due to space restrictions within the compression chamber, the tablet sensor is usually arranged in an area where tablets compressed in dies of the die disk are ejected from the dies, in particular pushed up by the lower punches, towards the top surface of the die disk. Once the tablets have reached the top surface of the die disk, they can be removed from the die disk and be guided to an exit of the compression chamber, for example by a scraper arranged above the die disk. This positional arrangement of the tablet sensor means that while the tablets pass through the measuring field of the tablet sensor together with the rotating die disk, the tablets also perform a movement in the axial direction of the die disk such that the tablets move relative to the focus of the tablet sensor. The tablet sensor comprises an emitter emitting electromagnetic radiation onto the upper surface of the tablet to be measured. For best measuring results it is desired that the electromagnetic radiation is focused onto the top surface of the tablets. Due to the explained axial movement of the tablets, this would only be possible by adapting the focus of the emitter which in practice is very complex. Therefore, in the prior art the emitter focuses the electromagnetic radiation on a mean axial positional value of the tablet surface. This also influences the measuring results and cannot be properly considered in a static calibration method as explained above.

Starting from the above explained prior art is an object of the present invention to provide a calibration device and method of the above explained type which enables a precise calibration of a tablet sensor for a tablet press in a simple, flexible and cost-efficient manner.

The invention solves this object on basis of the independent claims. Advantageous embodiments are the subject of the dependent claims, as well as the specification and the drawings.

For a calibration device of the type explained above, the invention solves the object, in that the calibration device comprises a tablet sensor configured to measure a content property of tablets, that the calibration device further comprises a calibration disk and a rotary drive for rotating the calibration disk about a rotational axis, wherein the calibration disk comprises a plurality of tablet receptacles, said tablet receptacles each configured to accommodate a pre-produced tablet with a known content property in a fixed position during rotation of the calibration disk, wherein the tablet sensor is arranged above the calibration disk such that tablets accommodated in the tablet receptacles rotate together with the calibration disk through a measuring field of the tablet sensor, wherein the tablet sensor obtains measuring data of a content property of the tablets in the measuring field, wherein the tablet sensor comprises an emitter, configured to emit electromagnetic measurement radiation onto an upper surface of a tablet in the measuring field, and a receiver, configured to receive electromagnetic measurement radiation reflected by the upper surface of the tablet, wherein a bottom surface of the tablet receptacles is formed such that the distance between the emitter and an upper surface of a tablet accommodated in a tablet receptacle decreases during rotation of the tablet through the measuring field of the tablet sensor, and in that the calibration device further comprises a control device, said control device receiving the measuring data from the tablet sensor, and being configured to calibrate the tablet sensor on basis of the received measuring data and the known property contents of the tablets.

For a method of the above explained type the invention solves the object by the steps:
- pre-produced tablets with a known content property are accommodated in a plurality of tablet receptacles of a calibration disk,
- the calibration disk is rotated about a rotational axis, wherein the tablets are in a fixed position in the tablet receptacles during rotation of the calibration disk,
- the tablet sensor is arranged above the calibration disk such that tablets accommodated in the tablet receptacles rotate together with the calibration disk through a measuring field of the tablet sensor,
- the tablet sensor obtains measuring data of a content property of the tablets in the measuring field, wherein the tablet sensor emits electromagnetic measurement radiation onto an upper surface of a tablet in the measuring field and receives electromagnetic measurement radiation reflected by the upper surface of the tablet, wherein the distance between the emitter and an upper surface of a tablet accommodated in the tablet receptacle decreases during rotation of the tablet through the measuring field of the tablet sensor,
- the tablet sensor is calibrated on basis of the measuring data and the known property contents of the tablets.

The inventive calibration device is not a (rotary) tablet press, but is arranged separately from a tablet press in which the tablet sensor to be calibrated shall be used during production. Such a tablet press may be a rotary tablet press. Consequently, the inventive calibration device in particular does not comprise a filling station or a pressing station or an ejector station as provided by a tablet press. Preferably, the calibration disk only comprises the tablet receptacles. The tablet receptacles are arranged such that tablets are held in a fixed position in the tablet receptacles during rotation, in particular during at least one full rotation, preferably multiple full rotations, of the calibration disk. In particular, tablets cannot fall through the receptacles during rotation of the calibration disk.

The tablet receptacles are adapted to the form of the tablets to be measured, in particular have a form complementary to the form of the tablets. The tablet receptacles are in particular formed as recesses or depressions in the calibration disk. The tablet receptacles are in particular not bores with an open bottom. Rather, the tablet receptacles are closed at the bottom such that tablets accommodated in the receptacles cannot exit the receptacles through the bottom. The calibration disk may for example comprise more than five receptacles, preferably at least 10 receptacles, more preferably more than 10 receptacles. The tablet receptacles are arranged along a circle on the calibration disk, preferably at regular intervals. The receptacles preferably have the same form and orientation on the calibration disk.

A rotary drive is provided which drives the calibration disk to rotate about a rotational axis, which may for example be a vertical rotational axis. During rotation, tablets remain in a fixed position in the tablet receptacles, as explained above. In particular, tablets are not moved in an axial direction parallel to the rotational axis of the calibration disk during rotation of the calibration disk. Due to this embodiment of the calibration disk, the exact position of the tablets to be measured is reliably defined such that the measuring trajectory of the tablet sensor on the surface of the tablets is the same for each tablet.

The tablets arranged in the tablet receptacles of the calibration disk are pre-produced, i.e. they are not produced in the calibration device. The pre-produced tablets used during the inventive calibration process may for example have been produced in a small batch press, such as an experimental press, or even in a press with only a single pair of an upper and lower punch.

The invention is based on the idea to provide a calibration device which can simulate the conditions within a tablet press precisely without the above explained disadvantages of calibrating the tablet sensor within the compression chamber of the tablet press. The rotating calibration disk with the tablets contained in the tablet receptacles simulates the rotating die disk of a tablet press, and thus the relative movement between the tablets to be measured and the tablet sensor as is present in the tablet press. Influences of further specific parameters and boundary conditions of the tablet press, which have to be controlled and documented in a complex matter as explained above, can be largely avoided. By providing a plurality of tablet receptacles in the calibration disk it is possible to obtain measuring data for a plurality of pre-produced tablets during rotation of the calibration disk. The obtaining of measuring data for several tablets is necessary for statistical methods used during the formation of a model for the specific product mixture used for pressing the tablets. For example, tablets with different known contents of a specific ingredient, for example an active pharmaceutical ingredient, can be placed in the tablet receptacles such that a content curve for different ingredient contents can be obtained based on the measuring data from the tablet sensor. Such content curves can be used during production in the actual tablet press for determining the specific ingredient content measured by the tablet sensor. As explained above, the overall weight of the tablets is usually known or can be measured in the production process of the tablet press. Based on the measurement values on the content of certain ingredients, also the weight of these ingredients in a tablet can be reliably determined on this basis.

The inventive calibration device and method also allow repeatedly measuring the same tablets during a calibration process, and thus further minimizing the product amount necessary for the calibration. The essential parameters influencing the measurement results, for example the distance between the tablet sensor and the tablet, the rotational speed of the tablet during measurement, as well as the measurement points on the surface of the tablet during rotation, can be reliably created in the calibration device.

The invention thus allows the calibration and the creation of a corresponding measuring model with a minimum amount of product. In extreme cases, a single tablet could be sufficient for the creation of a first model. In this way, the model creation for new products can be achieved considerably earlier in the development process. Obtaining early data accelerates reaching process safety for the production process and time to market as an essential factor for maximizing gains, for example since after expiry of patents, possible selling prices reduce considerably. The use of smaller amounts of product reduces development costs considerably, bearing in mind sometimes extremely expensive products. By reducing parameters influencing the measurement data to a minimum, the quality of the model and the calibration can be significantly increased. For example, influencing factors, such as a tolerance of the length and dimensions of press punches, the adjustment of an ejector, the expansion of a press frame or deviations in the filling system are completely avoided.

Due to the inventive feature of a bottom surface of the tablet receptacles, said bottom surface accommodating the tablet in the tablet receptacle, being formed such that the distance between the emitter of the tablet sensor and an upper surface of a tablet accommodated in a tablet receptacle decreases during rotation of the tablet through the measuring field of the tablet sensor, the above explained positional arrangement of the tablet sensor in in a compression chamber of a tablet press, where tablets perform an axial movement towards the emitter during their rotation through the measuring field, can also be precisely and reliably simulated. Any influences on the measuring data due to this axial movement and due to the related movement of the tablet with regard to the focal point of the emitter, can be reliably considered during calibration. As explained above, the tablet sensor and its focal point are in particular fixed and not adapted during movement of the tablet through the measuring field. Due to the decreasing distance between the emitter and the upper surface of the tablet during rotation through the measuring field a for example linear lift movement of a tablet during ejection from a die can be precisely and flexibly re-created in the calibration device and method. For example, by adapting the slope of the decrease, the calibration can be adapted to different press configurations and types.

Preferably the receiver receives electromagnetic radiation reflected by an upper surface of a tablet also along the main measuring direction. Accordingly, the electromagnetic radiation emitted by the emitter may have a perpendicular or normal angle of incidence on the upper surface of the tablet.

The calibration device is reduced to a necessary minimum and thus much cheaper than an actual tablet press. Calibration can be carried out in a shorter timeframe and is less complex such that costs are reduced. Furthermore, since for the inventive calibration process pre-produced tablets are used, dust creation can be avoided, which may be a hazard to operating personnel. The calibration device thus poses less demands regarding health and safety and can be used more flexibly. Also, as will be explained in more detail below, the invention allows calibration and model finding for a large variety of different types of tablet presses.

According to an embodiment, the distance between the emitter and the upper surface of a tablet accommodated in a tablet receptacle decreases evenly during rotation of the tablet through the measuring field of the tablet sensor. An even decrease of the distance simulates a linear upward movement of a tablet in a cavity of a disk die of a tablet press with a constant axial speed of movement.

As explained the emitter may be configured to emit electromagnetic measurement radiation onto an upper surface of a tablet in the measuring field along a main measuring direction. The main measuring direction may be tilted relative to the rotational axis of the calibration disk, preferably tilted by at least 3°, more prefearbly by at least 5°, more preferably tilted by at least 8°. The tilt angle may be less than 25°, preferably less than 20°. The additional vertical movement of a tablet in the measuring field of the tablet sensor in the compression chamber of a tablet press can be realized in the inventive compression device and method by tilting the main measuring direction of the emitter in relation to the rotational axis of the calibration disk. The emitter may emit electromagnetic radiation with a certain divergence and an angle of radiation. For example, the emitter may emit electromagnetic radiation with a cone shaped beam. As explained above, the electromagnetic radiation is though focused onto a focal point, which usually corresponds to the mean axial position of the upper surface of a tablet during its axial movement during ejection in the tablet press. For focusing, the tablet sensor may comprise corresponding optical elements. Consequently, the radiation emitted by the emitter may be focused on a mean value of the decreasing distance between the emitter and the upper surface of a tablet accommodated in a tablet receptacle of the calibration device. The main measuring direction accordingly extends from the emitter towards the focal point. By tilting the measuring coordination system of the tablet sensor - tablet system towards the coordination system of the movement of the tablet, the axial movement of a tablet in a tablet press can be reliably re-created. The tilting direction of the main measuring direction extends in particular in the direction of rotation of the calibration disk and its tablet receptacles. The tilting angle may be chosen to correspond to a gradient or slope of an ejector guide, in particular an ejector curve, arranged in the tablet press which cooperates with a punch head of the lower punches and pushes the lower punches upwards in a die to eject the tablets to the top of the die disk. The angle of tilt may in particular correspond to the gradient angle of such an ejector guide.

According to a further embodiment, adjustment means may be provided, said adjustment means being configured to adjust the tilt of the main measuring direction relative to the rotational axis of the calibration disk. The adjustment means may in particular be configured to tilt the tablet sensor. This embodiment allows for adjusting the calibration device to simulate different tablet press designs, in particular different gradients or slopes of an ejector element for ejecting tablets from dies of a die disk. The adjustment means may be controlled by the control device.

According to a further embodiment it is possible that the bottom surface of the tablet receptacles is formed such that the upper surface of tablets accommodated in the tablet receptacles is perpendicular to the main measuring direction at least along a trajectory of electromagnetic radiation emitted by the emitter onto the upper surface of the tablets, in particular a trajectory of a focal point of the emitter on the upper surface of the tablets, during rotation through the measuring field.

The tablet receptacles may have a form complementary to tablets to be accommodated therein. The tablets have an upper surface onto which the measuring trajectory of the tablet sensor, in particular the electromagnetic radiation emitted by the emitter, extends during measurement, i.e. during rotation of the tablet through the measuring field. This upper surface may be perpendicular to the main measuring direction at least in the area which is covered by the electromagnetic radiation, in particular a focal point, of the emitter during a measurement process. Thus, the electromagnetic radiation emitted onto the surface is reflected back towards the receiver also along the main measuring direction. The receiver may be arranged substantially at the same position as the emitter. Of course, they could also be arranged at different positions, wherein electromagnetic radiation may, for example, be guided from the emitter to the tablet and back from the tablet to the receiver through a beam splitter, such as a semi-transparent mirror or the like.

According to an embodiment the rotary drive may comprise an electric motor, in particular a servo motor. Such electric motors can be flexibly and individually controlled to enable simulating different rotor speeds of a tablet press. The control device may be configured to control the rotary drive to rotate the calibration disk at different rotational speeds for different calibration processes.

The content property may be a content of an active pharmaceutical ingredient (API) in the tablet. As explained above, tablets usually comprise one or more APIs and one or more excipients. Of course, it is necessary to precisely determine in particular the content of an API in a tablet.

The tablet sensor may be a spectroscopic sensor, preferably a NIR or LIF or Raman sensor. Such a sensor is particularly suited to measure for example an API content. Such sensors comprise an emitter emitting electromagnetic radiation, for example in the near infrared wavelength range, onto the surface of the tablet and a receiver receiving electromagnetic radiation reflected back by the surface of the tablet. The radiation may be focused on the surface of the tablet. By evaluating the spectrum of the reflected radiation, it is possible to determine the content of certain ingredients in the tablet.

The tablet sensor may be positioned on a calibration table, wherein the calibration table also accommodates the calibration disk. Also, the rotary drive may be arranged on or under the calibration table, for example held on the underside of the calibration table. The calibration table may be positioned in the same room as a tablet press to be equipped with the tablet sensor or separate from such a production room.

According to a further embodiment the tablet sensor may have attachment means configured to cooperate with attachment means of the calibration table, wherein the tablet sensor can be removably attached to the calibration table through cooperation of the attachment means of the calibration table with the attachment means of the tablet sensor. This allows for the tablet sensor to be easily installed in and removed from the calibration device. In particular, it allows to easily swap the tablet sensor between the calibration device and the tablet press.

The tablet sensor of the calibration device is preferably the same tablet sensor to be installed in the tablet press. This of course avoids potential influences of specific characteristics of a specific tablet sensor on the measuring data. According to a further embodiment the attachment means of the tablet sensor may be configured to cooperate with attachment means in a compression chamber of a tablet press such that the tablet sensor can also be removably attached in the compression chamber of the tablet press. In this manner the same tablet sensor to be used in the tablet press can be easily installed in the calibration device for calibration, and vice versa. Generally, the tablet sensor may comprise the same components as in the tablet press, for example components to hold and/or adjust the position of the tablet sensor. The inventive calibration device may thus be suited to be equipped with the full tablet sensor arrangement as is provided in the tablet press.

According to a further embodiment it is possible, that the calibration device comprises a plurality of different calibration disks, wherein the calibration disks have different diameters and/or wherein the calibration disks have different amounts and/or forms of tablet receptacles and/or tablet receptacles arranged on circles with different diameters, and in that the calibration disks are each provided with attachment means configured to cooperate with attachment means of the calibration table, wherein the calibration disks can alternatively be removably attached to the calibration table through cooperation of the attachment means of the calibration table with the attachment means of the calibration disks. This embodiment allows for flexible adaptation of the calibration device to simulate different types and configurations of tablet presses and their components, such as the rotor, as well as the tablets to be produced.

According to a further embodiment the control device may be configured to control the tablet sensor to start a measuring procedure when a tablet to be measured enters the measuring field of the tablet sensor and to terminate the measuring procedure when the tablet to be measured exits the measuring field of the tablet sensor. According to a further embodiment in this regard the calibration device may further comprise a rotational angle sensor, said rotational angle sensor providing the rotational angle of the calibration disk during rotation to the control device, wherein the control device is configured to control the tablet sensor to start and terminate the measuring procedure based on the rotational angle provided by the rotational angle sensor. The rotational angle sensor can be provided for example by an electric motor of the rotary drive which is able to provide the rotational angle of the calibration disk in certain defined steps. The rotational angle sensor may also be a separate sensor, arranged on the calibration disk or relative to the calibration disk and providing the rotational angle position of the calibration disk during rotation. The rotational angle sensor may for example comprise an angle encoder. According to the above embodiments the control device triggers the tablet sensor to start a measuring procedure when a tablet to be measured enters the measuring field, and to terminate measuring procedure when the tablet to be measured exits the measuring field. Based on these trigger events the tablet sensor then obtains measuring data along a measuring path or trajectory over the surface of the tablet corresponding to the rotation of the tablet together with the calibration disk through the measuring field. In this manner measuring data can be provided over the full extension of the tablet as it travels through the measuring field. The rotational angle sensor may be provided such that angle deviations towards the actual angle position of the calibration disk may be less than 0.8°, preferably less than 0.4°, more preferably less than 0.2°.

According to a further embodiment the calibration device may comprise position adjustment means, said position adjustment means being configured to adjust the position of the tablet sensor relative to the calibration disk in at least one spatial direction, preferably in at least two spatial directions, and more preferably in all three spatial directions. Being able to adjust the tablet sensor in this manner allows to reliably set a focus point of the tablet sensor relative to the surface of the tablet, for example in the vertical or z-direction. It also allows adjusting the position of the tablet sensor relative to different forms of tablets and/or different calibration disks having differently formed or arranged tablet receptacles. According to a further embodiment the control device may be configured to control the position adjustment means. This allows for an automatic adjustment of the position of the tablet sensor.

The calibration device may further comprise an optical reference surface, preferably a white reference surface, wherein the tablet sensor can be positioned such that the optical reference surface is positioned in the measuring field of the tablet sensor, and wherein the control device is configured to balance, in particular to white balance, the tablet sensor against the optical reference surface. The positioning of the reference surface in the measuring field may for example be achieved by adjusting the position of the tablet sensor with position adjustment means. This embodiment improves measuring data.

According to a further embodiment it is possible that the tablet receptacles are formed in receptacle holders, said receptacle holders being releasably fastened in the calibration disk, wherein the calibration device preferably comprises different sets of receptacle holders, which can be alternatively releasably fastened in the calibration disk, wherein the different sets of receptacle holders have differently formed tablet receptacles. In this manner the calibration device, in particular the calibration disk, can be easily adapted to different tablet forms. More specifically, if a different type of tablet shall be used for the calibration process the currently installed set of receptacle holders can be removed and replaced with a different set of receptacle holders having differently formed tablet receptacles. This allows using different types of tablets to be utilized in the calibration process without having to replace the calibration disk.

The invention also pertains to a system comprising a tablet press, in particular a rotary tablet press, and a calibration device according to the present invention.

According to a further embodiment it is possible that the tablet receptacles of the calibration disk are arranged along a circle with a first diameter, and in that cavities in a die disk of a rotor of the tablet press, in which cavities powder material is compressed to tablets by punches of the tablet press during production, are arranged along a circle with a second diameter, wherein the first diameter is smaller than the second diameter. More specifically, according to the above embodiment the calibration disk along with the circle of the tablet receptacles may be provided with a smaller diameter than the die disk of the rotor in the actual tablet press. It is nevertheless possible to achieve the same rotational speed of the calibration disk as a potentially larger die disk in a rotor. More specifically, since in the inventive calibration device and system no powder is being processed, but rather pre-produced tablets are used, losing powder from cavities due for example to centrifugal force, is not an issue. Using a smaller diameter calibration disk and circle of tablet receptacles also reduces height deviations of the surface of a calibration disk during rotation compared to larger diameter disks. Such height deviations are particularly critical for the measuring results. According to the invention height deviations of the upper surface of the calibration disk during a full rotation may be less than 1 mm, preferably less than 0.7 mm, more preferably less than 0.4 mm.

According to an embodiment of the inventive method it is possible that the tablets accommodated in the tablet receptacles each have the same content property, and in that the calibration disk is rotated by 360° for calibrating the tablet sensor or in that at least some of the tablets accommodated in the tablet receptacles, preferably all of the tablets accommodated in the tablet receptacles, have different content properties, and in that the calibration disk is rotated by 360° several times for calibrating the tablet sensor, preferably at least as often as there are tablets in the tablet receptacles with different content properties. As explained above it is possible to measure one tablet several times in the inventive calibration device and method by rotating the calibration disk by more than 360°, in particular several times for 360°. It is also possible to rotate the calibration disk only once for example by 360°. Depending on whether the tablet receptacles are fitted with tablets having the same content property or having different content properties it is thus possible to obtain sufficient statistical data for the calibration and building of a model.

The inventive calibration device or system may also comprise tablets accommodated in the tablet receptacles.

As already explained above, it is further possible that the decrease of the distance between the emitter and the upper surface of a tablet accommodated on the bottom surface during rotation of the tablet through the measuring field of the tablet sensor is approximated to, and preferably corresponds to, the decrease of the distance between the emitter of the tablet sensor and the upper surface of a tablet ejected to the upper surface of a die disk by a lower punch of the tablet press during rotation of the rotor of the tablet press, when the tablet sensor is installed in the tablet press. In this manner the axial movement of the tablets in the tablet press during ejection can be particularly precisely simulated.

The inventive method may be carried out with an inventive calibration device or an inventive system. Consequently, the inventive calibration device or the inventive system may be configured to carry out the inventive method.

An embodiment of the invention will be explained in more detail in the following with reference to the drawings. It is shown schematically in:
- Figure 1: a calibration device according to the invention in a perspective view,
- Figure 2: the calibration device shown in Figure 1 in a side view,
- Figure 3: the calibration device shown in Figure 1 in a top view,
- Figure 4: a sectional view along line B-B in Figure 3, and
- Figure 5: an inventive system comprising the calibration device shown in Figure 1 and a tablet press in a side view.

In the drawings the same reference numerals denote the same parts, unless specified otherwise.

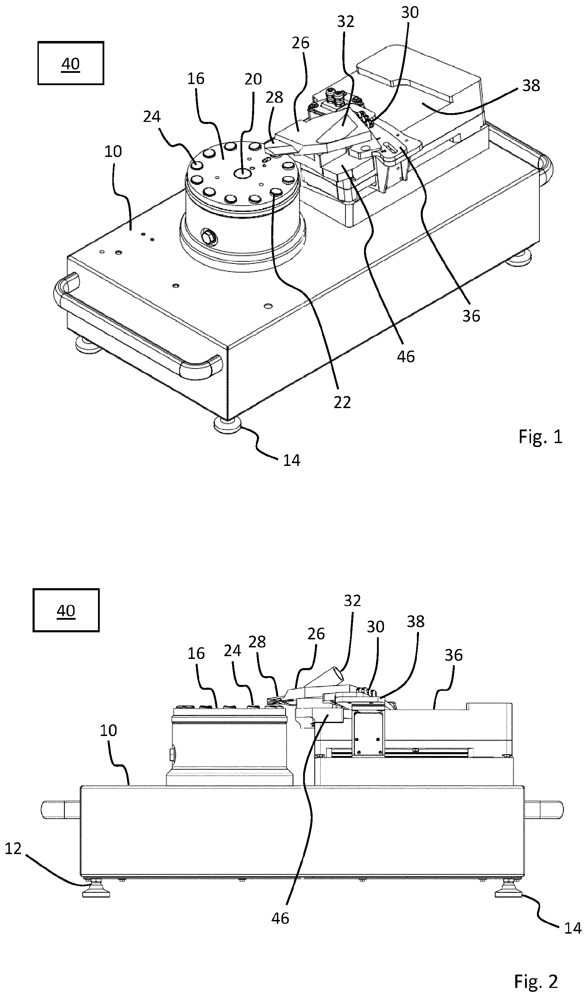

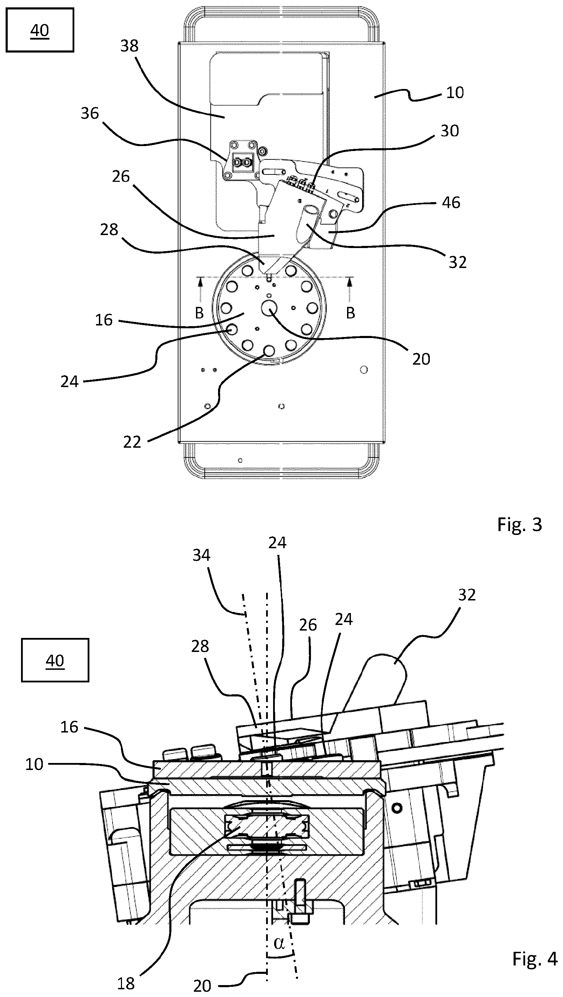

The inventive calibration device shown in Figures 1 to 3 comprises a calibration table 10 with, in the example shown, four legs 12 and feet 14 with which the calibration device can be placed on a floor. On the calibration table 10 a calibration disk 16 is positioned in a rotatable manner. A rotary drive 18, comprising for example an electric motor, such as a servo motor, is arranged underneath the calibration table 10 and can drive the calibration disk 16 to rotate about a rotational axis 20, which in the example shown is a vertical rotational axis 20. The calibration disk 16 comprises a plurality of tablet receptacles 22, each for accommodating a pre-produced tablet 24 in fixed position during rotation of the calibration disk 16. The tablet receptacles 22 each have the same form and are arranged in equal intervals in a circle in the calibration disk 16. As can be seen for example in the top view in Figure 3 the tablet receptacles 22, and thus the tablets 24 held in the tablet receptacles 22 are each of circular shape. The tablet receptacles 22 are formed as depressions in the calibration disk 16 with a closed bottom surface and have a form complementary to the form of the tablets.

The bottom surface of the tablet receptacles 22 is formed such that an upper surface of the tablets 24 is tilted relative to the upper surface of the calibration disk 16, as can be seen in particular in the sectional view of Figure 4. The tablets 24 may, for example, have a cylindrical shape with a flat top and bottom, wherein the bottom surface of the tablet receptacles 22 is tilted relative to the upper surface of the calibration disk 16 such that the tablets 24 are each accommodated in a tablet receptacle 22 in an inclined or slanting position.

The inventive calibration device further comprises a tablet sensor 26, in particular a spectroscopic sensor, like a NIR or LIF or Raman sensor, measuring a content property, such as an API content, of the tablets 24. To this end, a measuring head 28 comprising an emitter and a receiver for electromagnetic radiation is positioned above the calibration disk 16 such that the tablets 24 accommodated in the tablet receptacles 22 rotate together with the calibration disk 16 through a measuring field of tablet sensor 26, in particular of measuring head 28, and the tablet sensor 26 obtains measuring data of a content property of the tablets 24 in the measuring field. The tablet sensor 26 comprises several connections 30 for connecting for example optical fibers, as well as a suction connection 32 for connection to a suction device, for example a dust suction device for keeping the measuring field free of contaminations.

In the measuring head 28 of tablet sensor 26 an emitter is arranged which during a measurement process emits electromagnetic radiation, for example in the near-infrared wavelength region, onto an upper surface of a tablet 24 rotating through a measuring field of the tablet sensor 26 during rotation of the calibration disk 16. The electromagnetic radiation emitted by the emitter is focused in a focal point and emitted along a main measuring direction. In the example shown, and as can be seen in particular in Figure 4, tablet sensor 26 is tilted such that the main measuring direction of the radiation emitted by the emitter is correspondingly tilted relative to the rotational axis 20 of calibration disk 16. In the example shown, the main measuring direction is tilted such that electromagnetic radiation emitted by the emitter impinges with a perpendicular angle of incidence onto the upper surface of the slanted tablets 24 such that radiation reflected by the upper surface is reflected back to the measuring head 28 also along the main measuring direction, where it is received by a receiver of the tablet sensor 26. Due to the rotation of the calibration disk 16 and the above explained tilting, the distance between the emitter and the upper surface of a tablet 24 passing through the measuring field of the tablet sensor 26 decreases with the tablet 24 passing through the measuring field. In the example shown, the calibration disk 16 is rotated in a clockwise direction. Due to this arrangement a vertical movement of a tablet when being ejected from a die of a die disk in a tablet press towards the emitter of the tablet sensor arranged in the compression chamber can be reliably and precisely simulated. For better understanding, the rotational axis 20 as well as the main measuring direction 34 of the tablet sensor 26 are shown in Figure 4 together with the tilt angle α between the rotational axis 20 and the main measuring direction 34. The tilt angle may for example be at least 5°, preferably at least 8°.

Tablet sensor 26 is arranged on position adjustment means 36 arranged on an adjustment housing 38 which allow to adjust the position of the tablet sensor 26 in all three spatial directions. It is possible that the adjustment of the position is carried out manually by an operator. However, it is also possible that the position adjustment means 36 comprises at least one drive for adjusting the position of tablet sensor 26.

The inventive calibration device further comprises a control device 40 which may be configured to control the position adjustment means 38. Control device 40 may also be configured to control rotary drive 18 to rotate the calibration disk 16 during a calibration process. Measuring data obtained by tablet sensor 26 is provided to control device 40. Control device 40 is also provided with the known content property of the tablets 24 accommodated in the tablet receptacles 22, such that control device 40 can calibrate tablet sensor 26 based on the measuring data received and the known content properties of tablets 24. Control device 40 may further trigger tablet sensor 26 to start a measuring procedure when a tablet 24 to be measured enters the measuring field of the tablet sensor 26 and to terminate the measuring procedure when the tablet 24 exits the measuring field of the tablet sensor 26. To this end, the calibration device may be provided with a rotational angle sensor, which provides the rotational angle of the calibration disk 16 during rotation to the control device 40 such that the control device 40 controls tablet sensor 26 to start and terminate the measuring procedure based on the rotational angle provided by the rotational angle sensor. As explained above, the rotational angle sensor may, for example, be provided by rotary drive 18.

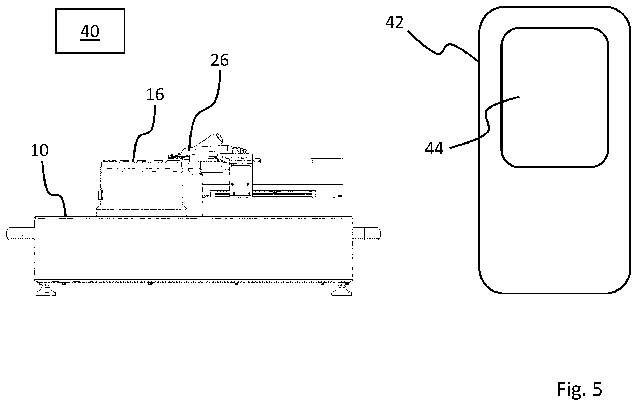

Tablet sensor 26 is further provided with attachment means which cooperate with attachment means of calibration table 10 such that tablet sensor 26 can be removably attached to calibration table 16 through cooperation of the attachment means. After calibration has been completed, tablet sensor 26 can thus be removed from calibration table 10 and can be mounted in a tablet press 42, as shown in the inventive system of Figure 5, in particular within the compression chamber 44 of tablet press 42. To this end, the attachment means of tablet sensor 26 can cooperate with corresponding attachment means within the compression chamber 44 of the tablet press 42.

It is possible that the tilted positioning of tablet sensor 26 is fixedly realized, for example by providing a holding plate 46 of the tablet sensor 26 with a tilted bottom surface such that when installed on the adjustment housing 38 the tablet sensor 26 is provided with the desired tilt. While not shown in the drawings, it would also be possible to provide adjustment means configured to adjust the tilt of tablet sensor 26. Such adjustment means could also be controlled by control device 40.

### Reference numerals

- 10: Calibration table
- 12: Legs
- 14: Feet
- 16: Calibration disk
- 18: Rotary drive
- 20: Rotational axis
- 22: Tablet receptacles
- 24: Tablets
- 26: Tablet sensor
- 28: Measuring head
- 30: Connections
- 32: Suction connection
- 34: Main measuring direction
- 36: Adjustment means
- 38: Adjustment housing
- 40: Control device
- 42: Tablet press
- 44: Compression chamber
- 46: Holding plate

## Claims

1. Calibration device for calibrating a tablet sensor (26) for a tablet press (42), **characterized in that** the calibration device (10) comprises a tablet sensor (26) configured to measure a content property of tablets (24), that the calibration device (10) further comprises a calibration disk (16) and a rotary drive (18) for rotating the calibration disk (16) about a rotational axis (20), wherein the calibration disk (16) comprises a plurality of tablet receptacles (22), said tablet receptacles (22) each configured to accommodate a pre-produced tablet (24) with a known content property in a fixed position during rotation of the calibration disk (16), wherein the tablet sensor (26) is arranged above the calibration disk (16) such that tablets (24) accommodated in the tablet receptacles (22) rotate together with the calibration disk (16) through a measuring field of the tablet sensor (26), wherein the tablet sensor (26) obtains measuring data of a content property of the tablets (24) in the measuring field, wherein the tablet sensor (26) comprises an emitter, configured to emit electromagnetic measurement radiation onto an upper surface of a tablet (24) in the measuring field, and a receiver, configured to receive electromagnetic measurement radiation reflected by the upper surface of the tablet (24), wherein a bottom surface of the tablet receptacles (22) is formed such that the distance between the emitter and an upper surface of a tablet (24) accommodated in a tablet receptacle (22) decreases during rotation of the tablet (24) through the measuring field of the tablet sensor (26), and **in that** the calibration device (10) further comprises a control device (40), said control device (40) receiving the measuring data from the tablet sensor (26), and being configured to calibrate the tablet sensor (26) on basis of the received measuring data and the known property contents of the tablets (24).

2. Calibration device according to claim 1, **characterized in that** the distance between the emitter and the upper surface of a tablet accommodated in a tablet receptacle (22) decreases evenly during rotation of the tablet through the measuring field of the tablet sensor (26).

3. Calibration device according to one of the preceding claims, **characterized in that** the emitter is configured to emit electromagnetic measurement radiation onto an upper surface of a tablet (24) in the measuring field along a main measuring direction (34), and **in that** the main measuring direction (34) is tilted relative to the rotational axis (20) of the calibration disk (16), preferably tilted by at least 3°, more preferably tilted by at least 5°, and more preferably tilted by at least 8°.

4. Calibration device according to claim 3, **characterized in that** adjustment means are provided, said adjustment means being configured to adjust the tilt of the main measuring direction (34) relative to the rotational axis (20) of the calibration disk (16).

5. Calibration device according to claim 4, **characterized in that** the adjustment means are configured to tilt the tablet sensor (26).

6. Calibration device according to one of the preceding claims, **characterized in that** the bottom surface of the tablet receptacles (22) is formed such that the upper surface of tablets accommodated in the tablet receptacles (22) is perpendicular to the main measuring direction (34) at least along a trajectory of electromagnetic radiation emitted by the emitter on the upper surface of the tablets (24) during rotation through the measuring field.

7. Calibration device according to one of the preceding claims, **characterized in that** the content property is a content of an active pharmaceutical ingredient in the tablet (24).

8. Calibration device according to one of the preceding claims, **characterized in that** the tablet sensor (26) is a spectroscopic sensor, preferably a NIR or LIF or Raman sensor.

9. Calibration device according to one of the preceding claims, **characterized in that** the tablet sensor (26) is positioned on a calibration table (10), wherein the calibration table (10) also accommodates the calibration disk (16), wherein the tablet sensor (26) has attachment means configured to cooperate with attachment means of the calibration table (10), wherein the tablet sensor (26) can be removably attached to the calibration table (10) through cooperation of the attachment means of the calibration table (10) with the attachment means of the tablet sensor (26).

10. Calibration device according to claim 9, **characterized in that** the attachment means of the tablet sensor (26) are configured to cooperate with attachment means in a compression chamber (44) of a tablet press (42) such that the tablet sensor (26) can also be removably attached in the compression chamber (44) of the tablet press. (42)

11. Calibration device according to one of the preceding claims, **characterized in that** it also comprises tablets (24) accommodated in the tablet receptacles (22).

12. System comprising a tablet press (42) and a calibration device according to one of the preceding claims.

13. System according to claim 12, **characterized in that** the decrease of the distance between the emitter and the upper surface of a tablet (24) accommodated on the bottom surface during rotation of the tablet through the measuring field of the tablet sensor (26) is approximated to, and preferably corresponds to, the decrease of the distance between the emitter of the tablet sensor (26) and the upper surface of a tablet (24) ejected to the upper surface of a die disk by a lower punch of the tablet press (42) during rotation of the rotor of the tablet press (42), when the tablet sensor (26) is installed in the tablet press (42).

14. Method for calibrating a tablet sensor (26) for a tablet press (42), said tablet sensor (26) being configured to measure a content property of tablets (24), **characterized by** the steps:
• pre-produced tablets (24) with a known content property are accommodated in a plurality of tablet receptacles (22) of a calibration disk (16),
• the calibration disk (16) is rotated about a rotational axis (20), wherein the tablets (24) are in a fixed position in the tablet receptacles (22) during rotation of the calibration disk (16),
• the tablet sensor (26) is arranged above the calibration disk (16) such that tablets (24) accommodated in the tablet receptacles (22) rotate together with the calibration disk (16) through a measuring field of the tablet sensor (26),
• the tablet sensor (26) obtains measuring data of a content property of the tablets (24) in the measuring field, wherein the tablet sensor (26) emits electromagnetic measurement radiation onto an upper surface of a tablet (24) in the measuring field and receives electromagnetic measurement radiation reflected by the upper surface of the tablet (24), wherein the distance between the emitter and an upper surface of a tablet (24) accommodated in the tablet receptacle (22) decreases during rotation of the tablet (24) through the measuring field of the tablet sensor (26),
• the tablet sensor (26) is calibrated on basis of the measuring data and the known property contents of the tablets (24).

15. Method according to claim 14, **characterized in that** it is carried out with a calibration device according to one of claims 1 to 11 or with a system according to one of claims 12 or 13.

## Patentansprüche

1. Kalibriervorrichtung zum Kalibrieren eines Tablettensensors (26) für eine Tablettenpresse (42), **dadurch gekennzeichnet, dass** die Kalibriervorrichtung (10) einen Tablettensensor (26) umfasst, der konfiguriert ist, um eine Inhaltseigenschaft von Tabletten (24) zu messen, dass die Kalibriervorrichtung (10) ferner eine Kalibrierscheibe (16) und einen Drehantrieb (18) zum Drehen der Kalibrierscheibe (16) um eine Drehachse (20) umfasst, wobei die Kalibrierscheibe (16) eine Mehrzahl von Tablettenaufnahmen (22) umfasst, wobei die Tablettenaufnahmen (22) jeweils konfiguriert sind, um eine vorproduzierte Tablette (24) mit einer bekannten Inhaltseigenschaft in einer festen Position während der Drehung der Kalibrierscheibe (16) aufzunehmen, wobei der Tablettensensor (26) oberhalb der Kalibrierscheibe (16) derart angeordnet ist, dass in den Tablettenaufnahmen (22) aufgenommene Tabletten (24) zusammen mit der Kalibrierscheibe (16) durch ein Messfeld des Tablettensensors (26) rotieren, wobei der Tablettensensor (26) Messdaten einer Inhaltseigenschaft der Tabletten (24) in dem Messfeld erhält, wobei der Tablettensensor (26) einen Sender umfasst, der konfiguriert ist, um elektromagnetische Messstrahlung auf eine obere Oberfläche einer Tablette (24) in dem Messfeld zu emittieren, und einen Empfänger, der konfiguriert ist, um elektromagnetische Messstrahlung zu empfangen, die von der oberen Oberfläche der Tablette (24) reflektiert wird, wobei eine Bodenfläche der Tablettenaufnahmen (22) derart geformt ist, dass der Abstand zwischen dem Sender und einer oberen Oberfläche einer in einer Tablettenaufnahme (22) aufgenommenen Tablette (24) während der Drehung der Tablette (24) durch das Messfeld des Tablettensensors (26) abnimmt, und dass die Kalibriervorrichtung (10) ferner eine Steuervorrichtung (40) umfasst, wobei die Steuervorrichtung (40) die Messdaten von dem Tablettensensor (26) empfängt und konfiguriert ist, um den Tablettensensor (26) auf Basis der empfangenen Messdaten und der bekannten Inhaltseigenschaften der Tabletten (24) zu kalibrieren.

2. Kalibriervorrichtung nach Anspruch 1, **dadurch gekennzeichnet, dass** der Abstand zwischen dem Sender und der oberen Oberfläche einer in einer Tablettenaufnahme (22) aufgenommenen Tablette während der Drehung der Tablette durch das Messfeld des Tablettensensors (26) gleichmäßig abnimmt.

3. Kalibriervorrichtung nach einem der vorhergehenden Ansprüche, **dadurch gekennzeichnet, dass** der Sender konfiguriert ist, um elektromagnetische Messstrahlung auf eine obere Oberfläche einer Tablette (24) in dem Messfeld entlang einer Hauptmessrichtung (34) zu emittieren, und dass die Hauptmessrichtung (34) relativ zur Drehachse (20) der Kalibrierscheibe (16) geneigt ist, vorzugsweise um mindestens 3° geneigt, weiter vorzugsweise um mindestens 5° geneigt, und weiter vorzugsweise um mindestens 8° geneigt.

4. Kalibriervorrichtung nach Anspruch 3, **dadurch gekennzeichnet, dass** Einstellmittel vorgesehen sind, wobei die Einstellmittel konfiguriert sind, um die Neigung der Hauptmessrichtung (34) relativ zur Drehachse (20) der Kalibrierscheibe (16) einzustellen.

5. Kalibriervorrichtung nach Anspruch 4, **dadurch gekennzeichnet, dass** die Einstellmittel konfiguriert sind, um den Tablettensensor (26) zu neigen.

6. Kalibriervorrichtung nach einem der vorhergehenden Ansprüche, **dadurch gekennzeichnet, dass** die Bodenfläche der Tablettenaufnahmen (22) derart geformt ist, dass die obere Oberfläche von in den Tablettenaufnahmen (22) aufgenommenen Tabletten senkrecht zur Hauptmessrichtung (34) ist, zumindest entlang einer Trajektorie von elektromagnetischer Strahlung, die von dem Sender auf die obere Oberfläche der Tabletten (24) während der Rotation durch das Messfeld emittiert wird.

7. Kalibriervorrichtung nach einem der vorhergehenden Ansprüche, **dadurch gekennzeichnet, dass** die Inhaltseigenschaft ein Gehalt eines aktiven pharmazeutischen Wirkstoffs in der Tablette (24) ist.

8. Kalibriervorrichtung nach einem der vorhergehenden Ansprüche, **dadurch gekennzeichnet, dass** der Tablettensensor (26) ein spektroskopischer Sensor ist, vorzugsweise ein NIR- oder LIF- oder Raman-Sensor.

9. Kalibriervorrichtung nach einem der vorhergehenden Ansprüche, **dadurch gekennzeichnet, dass** der Tablettensensor (26) auf einem Kalibriertisch (10) positioniert ist, wobei der Kalibriertisch (10) auch die Kalibrierscheibe (16) aufnimmt, wobei der Tablettensensor (26) Befestigungsmittel aufweist, die konfiguriert sind, um mit Befestigungsmitteln des Kalibriertisches (10) zusammenzuwirken, wobei der Tablettensensor (26) durch Zusammenwirken der Befestigungsmittel des Kalibriertisches (10) mit den Befestigungsmitteln des Tablettensensors (26) lösbar an dem Kalibriertisch (10) befestigt werden kann.

10. Kalibriervorrichtung nach Anspruch 9, **dadurch gekennzeichnet, dass** die Befestigungsmittel des Tablettensensors (26) konfiguriert sind, um mit Befestigungsmitteln in einer Kompressionskammer (44) einer Tablettenpresse (42) derart zusammenzuwirken, dass der Tablettensensor (26) auch lösbar in der Kompressionskammer (44) der Tablettenpresse (42) befestigt werden kann.

11. Kalibriervorrichtung nach einem der vorhergehenden Ansprüche, **dadurch gekennzeichnet, dass** sie auch Tabletten (24) umfasst, die in den Tablettenaufnahmen (22) aufgenommen sind.

12. System, umfassend eine Tablettenpresse (42) und eine Kalibriervorrichtung nach einem der vorhergehenden Ansprüche.

13. System nach Anspruch 12, **dadurch gekennzeichnet, dass** die Abnahme des Abstands zwischen dem Sender und der oberen Oberfläche einer auf der Bodenfläche aufgenommenen Tablette (24) während der Drehung der Tablette durch das Messfeld des Tablettensensors (26) angenähert ist an, und vorzugsweise entspricht, der Abnahme des Abstands zwischen dem Sender des Tablettensensors (26) und der oberen Oberfläche einer Tablette (24), die durch einen Unterstempel der Tablettenpresse (42) während der Drehung des Rotors der Tablettenpresse (42) zu der oberen Oberfläche einer Matrizenscheibe ausgeworfen wird, wenn der Tablettensensor (26) in der Tablettenpresse (42) installiert ist.

14. Verfahren zum Kalibrieren eines Tablettensensors (26) für eine Tablettenpresse (42), wobei der Tablettensensor (26) konfiguriert ist, um eine Inhaltseigenschaft von Tabletten (24) zu messen, **gekennzeichnet durch** die Schritte:
• vorproduzierte Tabletten (24) mit einer bekannten Inhaltseigenschaft werden in einer Mehrzahl von Tablettenaufnahmen (22) einer Kalibrierscheibe (16) aufgenommen,
• die Kalibrierscheibe (16) wird um eine Drehachse (20) gedreht, wobei die Tabletten (24) während der Drehung der Kalibrierscheibe (16) in einer festen Position in den Tablettenaufnahmen (22) sind,
• der Tablettensensor (26) wird oberhalb der Kalibrierscheibe (16) derart angeordnet, dass in den Tablettenaufnahmen (22) aufgenommene Tabletten (24) zusammen mit der Kalibrierscheibe (16) durch ein Messfeld des Tablettensensors (26) rotieren,
• der Tablettensensor (26) erhält Messdaten einer Inhaltseigenschaft der Tabletten (24) in dem Messfeld, wobei der Tablettensensor (26) elektromagnetische Messstrahlung auf eine obere Oberfläche einer Tablette (24) in dem Messfeld emittiert und elektromagnetische Messstrahlung empfängt, die von der oberen Oberfläche der Tablette (24) reflektiert wird, wobei der Abstand zwischen dem Sender und einer oberen Oberfläche einer in der Tablettenaufnahme (22) aufgenommenen Tablette (24) während der Drehung der Tablette (24) durch das Messfeld des Tablettensensors (26) abnimmt,
• der Tablettensensor (26) wird auf Basis der Messdaten und der bekannten Inhaltseigenschaften der Tabletten (24) kalibriert.

15. Verfahren nach Anspruch 14, **dadurch gekennzeichnet, dass** es mit einer Kalibriervorrichtung nach einem der Ansprüche 1 bis 11 oder mit einem System nach einem der Ansprüche 12 oder 13 durchgeführt wird.

## Revendications

1. Dispositif d'étalonnage permettant d'étalonner un capteur de comprimés (26) pour une presse à comprimés (42), **caractérisé en ce que** le dispositif d'étalonnage (10) comporte un capteur de comprimés (26) configuré pour mesurer une propriété de teneur de comprimés (24), **en ce que** le dispositif d'étalonnage (10) comporte en outre un disque d'étalonnage (16) et un entraînement rotatif (18) pour faire tourner le disque d'étalonnage (16) autour d'un axe de rotation (20), dans lequel le disque d'étalonnage (16) comporte une pluralité de réceptacles de comprimés (22), lesdits réceptacles de comprimés (22) étant chacun conçu pour loger un comprimé (24) pré-produit avec une propriété de teneur connue dans une position fixe pendant la rotation du disque d'étalonnage (16), dans lequel le capteur de comprimés (26) est agencé au-dessus du disque d'étalonnage (16) de sorte que des comprimés (24) logés dans les réceptacles de comprimés (22) tournent avec le disque d'étalonnage (16) à travers un champ de mesure du capteur de comprimés (26), dans lequel le capteur de comprimés (26) obtient des données de mesure d'une propriété de teneur des comprimés (24) dans le champ de mesure, dans lequel le capteur de comprimés (26) comporte un émetteur, configuré pour émettre un rayonnement de mesure électromagnétique sur une surface supérieure d'un comprimé (24) dans le champ de mesure, et un récepteur, configuré pour recevoir un rayonnement de mesure électromagnétique réfléchi par la surface supérieure du comprimé (24), dans lequel une surface de fond des réceptacles de comprimés (22) est formée de sorte que la distance entre l'émetteur et une surface supérieure d'un comprimé (24) logé dans un réceptacle de comprimés (22) diminue pendant la rotation du comprimé (24) à travers le champ de mesure du capteur de comprimés (26), et **en ce que** le dispositif d'étalonnage (10) comporte en outre un dispositif de commande (40), ledit dispositif de commande (40) recevant les données de mesure de la part du capteur de comprimés (26) et étant configuré pour étalonner le capteur de comprimés (26) sur la base des données de mesure reçues et de la teneur de propriété connue des comprimés (24).

2. Dispositif d'étalonnage selon la revendication 1, **caractérisé en ce que** la distance entre l'émetteur et la surface supérieure d'un comprimé logé dans un réceptacle de comprimés (22) diminue de manière régulière pendant la rotation du comprimé à travers le champ de mesure du capteur de comprimés (26).

3. Dispositif d'étalonnage selon l'une des revendications précédentes, **caractérisé en ce que** l'émetteur est configuré pour émettre un rayonnement de mesure électromagnétique sur une surface supérieure d'un comprimé (24) dans le champ de mesure le long d'une direction de mesure principale (34), et **en ce que** la direction de mesure principale (34) est inclinée par rapport à l'axe de rotation (20) du disque d'étalonnage (16), de préférence inclinée d'au moins 3°, plus préférablement inclinée d'au moins 5° et le plus préférablement inclinée d'au moins 8°.

4. Dispositif d'étalonnage selon la revendication 3, **caractérisé en ce qu'**un moyen d'ajustement est prévu, ledit moyen d'ajustement étant configuré pour ajuster l'inclinaison de la direction de mesure principale (34) par rapport à l'axe de rotation (20) du disque d'étalonnage (16).

5. Dispositif d'étalonnage selon la revendication 4, **caractérisé en ce que** le moyen d'ajustement est configuré pour incliner le capteur de comprimés (26).

6. Dispositif d'étalonnage selon l'une des revendications précédentes, **caractérisé en ce que** la surface de fond des réceptacles de comprimés (22) est formée de sorte que la surface supérieure de comprimés logés dans les réceptacles de comprimés (22) soit perpendiculaire à la direction de mesure principale (34) au moins le long d'une trajectoire d'un rayonnement électromagnétique émis par l'émetteur sur la surface supérieure des comprimés (24) pendant la rotation à travers le champ de mesure.

7. Dispositif d'étalonnage selon l'une des revendications précédentes, **caractérisé en ce que** la propriété de teneur est une teneur d'un ingrédient pharmaceutique actif dans le comprimé (24).

8. Dispositif d'étalonnage selon l'une des revendications précédentes, **caractérisé en ce que** le capteur de comprimés (26) est un capteur spectroscopique, de préférence un capteur NIR ou LIF ou Raman.

9. Dispositif d'étalonnage selon l'une des revendications précédentes, **caractérisé en ce que** le capteur de comprimés (26) est positionné sur une table d'étalonnage (10), dans lequel la table d'étalonnage (10) loge également le disque d'étalonnage (16), dans lequel le capteur de comprimés (26) a un moyen d'attache conçu pour coopérer avec un moyen d'attache de la table d'étalonnage (10), dans lequel le capteur de comprimés (26) peut être attaché de manière amovible à la table d'étalonnage (10) à travers la coopération du moyen d'attache de la table d'étalonnage (10) avec le moyen d'attache du capteur de comprimés (26).

10. Dispositif d'étalonnage selon la revendication 9, **caractérisé en ce que** le moyen d'attache du capteur de comprimés (26) est configuré pour coopérer avec le moyen d'attache dans une chambre de compression (44) d'une presse à comprimés (42), de sorte que le capteur de comprimés (26) puisse être attaché de manière amovible dans la chambre de compression (44) de la presse à comprimés (42).

11. Dispositif d'étalonnage selon l'une des revendications précédentes, **caractérisé en ce qu'**il comporte également des comprimés (24) logés dans les réceptacles de comprimés (22).

12. Système comportant une presse à comprimés (42) et un dispositif d'étalonnage selon l'une des revendications précédentes.

13. Système selon la revendication 12, **caractérisé en ce que** la diminution de la distance entre l'émetteur et la surface supérieure d'un comprimé (24) logé sur la surface de fond pendant la rotation du comprimé à travers le champ de mesure du capteur de comprimés (26) est approximée à - et de préférence correspond à - la diminution de la distance entre l'émetteur du capteur de comprimés (26) et la surface supérieure d'un comprimé (24) éjecté à la surface supérieure d'un disque à matrice par une perforation inférieure de la presse à comprimés (42) pendant la rotation du rotor de la presse à comprimés (42) lorsque le capteur de comprimés (26) est installé dans la presse à comprimés (42).

14. Procédé d'étalonnage d'un capteur de comprimés (26) pour une presse à comprimés (42), ledit capteur de comprimés (26) étant configuré pour mesurer une propriété de teneur de comprimés (24), **caractérisé par** les étapes suivantes :
• des comprimés (24) pré-produits avec une propriété de teneur connue sont logés dans une pluralité de réceptacles de comprimés (22) d'un disque d'étalonnage (16),
• le disque d'étalonnage (16) est mis en rotation autour d'un axe de rotation (20), dans lequel les comprimés (24) sont dans une position fixe dans les réceptacles de comprimés (22) pendant la rotation du disque d'étalonnage (16),
• le capteur de comprimés (26) est agencé au-dessus du disque d'étalonnage (16), de sorte que des comprimés (24) logés dans les réceptacles de comprimés (22) tournent avec le disque d'étalonnage (16) à travers un champ de mesure du capteur de comprimés (26),
• le capteur de comprimés (26) obtient des données de mesure d'une propriété de teneur des comprimés (24) dans le champ de mesure, dans lequel le capteur de comprimés (26) émet un rayonnement de mesure électromagnétique sur une surface supérieure d'un comprimé (24) dans le champ de mesure et reçoit un rayonnement de mesure électromagnétique réfléchi par la surface supérieure du comprimé (24), dans lequel la distance entre l'émetteur et une surface supérieure d'un comprimé (24) logé dans le réceptacle de comprimés (22) diminue pendant la rotation du comprimé (24) à travers le champ de mesure du capteur de comprimés (26),
• le capteur de comprimés (26) est étalonné sur la base des données de mesure et de la teneur de propriété connue des comprimés (24).

15. Procédé selon la revendication 14, **caractérisé en ce qu'**il est réalisé avec un dispositif d'étalonnage selon l'une des revendications 1 à 11 ou avec un système selon l'une des revendications 12 ou 13.
